# EUROPEAN PATENT SPECIFICATION

(11) **EP 1 569 606 B1**
(45) Date of publication and mention of the grant of the patent: **11.11.2015**
(21) Application number: 03790314.3
(22) Date of filing: 05.12.2003
(51) Int. Cl.: A61Q 1/10, A61Q 5/06, A61Q 5/12, A61K 8/66

(54) **METHOD OF CURL RETENTION IN HAIR AND LASHES**
VERFAHREN FÜR DEN ERHALT VON LOCKEN IN HAAR UND WIMPERN
PROCEDE DE MAINTIEN DE BOUCLES DANS LES CHEVEUX ET DANS LES CILS

(30) Priority: 05.12.2002 US 431164 P
(43) Date of publication of application: 07.09.2005
(73) Proprietor: E-L Management Corp., New York, NY 10153 (US)
(72) Inventor: MAMMONE, Thomas, Farmingdale, NY 11735 (US); POPESCU, Lavinia, C., Jackson Heights, NY 11372 (US)
(74) Representative: Hirsch & Associés
(86) International application number: PCT/US2003/038502
(87) International publication number: WO 2004/052279

(56) References cited:
- US-A- 5 490 980
- US-A- 5 525 336
- US-B2- 6 399 052
- GARDNER J.M. ET AL.: "An investigation into the action of transglutaminase on human hair" J.SOC.COSMET.CHEM., vol. 46, January 1995 (1995-01), pages 11-28, XP000929527
- DATABASE WPI Week 1991 Derwent Publications Ltd., London, GB; AN 1991-320726 XP002353854 "Modification of animal hait fibre-by using enzyme to crosslink cuticle part of animal hair fibre" & JP 03 213574 A (AJINOMOTO KK) 18 September 1991 (1991-09-18)
- DATABASE WPI Week 1991 Derwent Publications Ltd., London, GB; AN 1991-159945 XP002353855 "Hair cosmetic material improving water retention and giving softness - contains trans-glutaminase modified with water soluble substance, e.g. polyethylene glycol" & JP 03 095109 A (KANEBO LTD) 19 April 1991 (1991-04-19)
- DATABASE WPI Week 1997 Derwent Publications Ltd., London, GB; AN 1997-294819 XP002353856 & JP 09 110647 A (LION CORP) 28 April 1997 (1997-04-28)
- PATENT ABSTRACTS OF JAPAN & JP 03 083908 A (KANEBO LTD), 9 April 1991 (1991-04-09)
- DATABASE WPI Week 1991 Derwent Publications Ltd., London, GB; AN 1991-092235 XP002353857 "Agent for external application to skin or hair-includes transglutaminase, can be in form of cream, emulsion, ointment or powder, give sgood conditioning effect" & JP 03 038511 A (POLA CHEM IND INC) 19 February 1991 (1991-02-19)
- DATABASE WPI Week 199038, Derwent Publications Ltd., London, GB; AN 1990-287814, XP002160503 'Hair cosmetic material compsn. - contg. trans:glutaminase, watersoluble poly:hydric alcohol and opt. calcium salt' & JP 2 204 407 A (KANEBO LTD) 14 August 1990
- John M Connellan ET AL: "Structural properties of guinea pig liver transglutaminase", J. Biol. Chem., 1 January 1971 (1971-01-01), pages 1093-1098, XP055140560, Retrieved from the Internet: URL:http://www.jbc.org/content/246/4/1093. full.pdf [retrieved on 2014-09-16]

## Description

### Field of the Invention

The invention discloses methods for treating hair and eyelashes. More specifically, the invention relates to a method for retaining curl in hair and lashes.

### Background of the Invention

As anyone with wave or curl (natural or otherwise) in his or her hair instinctively knows, dampness and humidity are anathema to a kempt hairstyle. It is so widespread a phenomenon that a damp, rainy day has acquired a popular culture definition as a "bad hair day". The technical explanation for the effect is well known. The disruption of the curl is the result of disruption of one or more bonds in the protein structure of the hair. Hair contains three primary bonds: hydrogen bonds(the weakest), which are affected by moisture; salt-peptide linkages (hydrophobic interaction of the aliphatic amino acid side chains, which are affected by moisture and heat; and cystine/cysteine bonds which to a large degree is responsible for the toughness/resilience of the hair. That hair is remarkably resilient is largely due to the presence of these bonds, which act as the main stabilizing forces in the hair. It has previously been shown that humidity increases the length, volume (swelling), and diameter of the hair strands. Heat may also have a swelling effect on the hair. These effects in turn affect the mechanical frictional properties (i.e., bending or elasticity) of the hair. As curly hair is subjected to both moisture and heat, the bonds (salt and hydrogen) are adversely affected to the point in which the hair swells, and increases in length, thereby causing a "curl droop".

That there is a technical explanation is of little consolation to those individuals who routinely suffer from the "frizzies" when a modicum of humidity is present in the air. There is also little in the way of relief for victims of this humidity-induced disorder of the tresses. A frequent solution is the use of styling gels that attempt to hold the curl in by the use of film forming polymers. Products of this type have variable efficacy, however. A truly effective product will frequently leave the hair stiff, sticky, and unnatural-feeling, while a more natural feeling product will ordinarily not stand up to severe weather conditions. There thus continues to be a need for a curl retention product that will not only permit curly hair to stand up to moisture, but also that can leave the hair feeling soft and natural.

### Summary of the Invention

The present invention relates to a method of retaining curl in keratinous material comprising applying to the keratinous material a composition containing a transglutaminase product in an amount ranging from 0.5% to 1.75% by weight, wherein said transglutaminase product is a combination of powdered microbial enzyme and maltodextrin containing 86-135 units of transglutaminase activity per gram. The present invention also relates to a method of retaining curl in a keratinous material which comprises applying to the keratinous material a composition containing transglutaminase in an amount sufficient to provide the composition with 0.4 - 2.3625 units of TGase activity per gram of composition.

### Detailed Description of the Invention

Transglutaminases are a family of enzymes that catalyze the posttranslational modification of proteins by transamidation of available glutamine residues. The major result is glutamyl-lysine crosslinks in proteins. These enzymes are found naturally throughout the body, including the hair and nails. A number of topical uses for transglutaminases have been proposed. JP 2719166 discloses compositions containing transglutaminase and a polyhydric alcohol, said to be useful in treatment of damaged hair by increasing the moisture retention of the hair. JP 3083908 suggests the use of transglutaminase in combination with polyethylene glycol or other water soluble materials to treat chapped skin. It has also been suggested for use in binding active components to skin, hair or nails(US 5,490,980). WO01/21145 teaches the use of transglutaminase to improve the color-fastness of hair dyes. WO01/21139 suggests a combination of transglutaminase and an active substance having substrate activity for transglutaminase, for use in restructuring damaged keratin fibers. US Patent No. 5,525,336 discloses the combination of corneocyte proteins and transglutaminase for application to skin, hair or nails to form a protective layer.

JP 2204407 discloses the use of transglutaminase to modify the surface of damaged hair to improve moisture- and water-retainability and impart gloss, softness and springiness.

To the best of Applicants' knowledge, transglutaminase has not previously been disclosed or used for curl retention in keratinous materials such as hair or eyelashes. Unexpectedly, when curly hair treated with a transglutaminase containing composition is exposed to high humidity, it retains a curl substantially better than untreated hair under the same conditions (see example 2). This result occurs at relatively low concentrations of active material and the effect is retained over a period of several hours, even if the material is first washed off. While not wishing to be bound by any theory, it is believed that the transglutaminase, in forming a crosslink between lysine and glutamine, two of the more common amino acids in hair, creates a covalent bond that is much more resistant to disruption by heat and humidity than are hydrogen or ionic bonds. The exposure of transglutaminase-treated hair to levels of moisture typically found atmospherically results in little or no lengthening (i.e., straightening) of the hair ("curl droop").

The present invention relates to a method of retaining curl in keratinous material comprising applying to the keratinous material a composition containing a transglutaminase product in an amount ranging from 0.5% to 1.75% by weight, wherein said transglutaminase product is a combination of powdered microbial enzyme and maltodextrin containing 86-135 units of transglutaminase activity per gram. The transglutaminase utilized in the present invention can be any transglutaminase from any source. Available sources of transglutaminase include, but are not limited to, slime mold, alfalfa, guinea pig, and bacteria, such as *Bacillus subtilis* or *Streptoverticillium.* The "curl retention effective amount" of transglutaminase used in a curling product in weight percent terms may vary depending upon the identity of the material, as different sources may have different enzyme potencies. One preferred form of transglutaminase is available from Ajinomoto USA (Ames, IA) under the trade name Activa™TG_TI. This product is a combination of powdered microbial enzyme and maltodextrin containing 86-135 units of transglutaminase activity per gram (.86-1.35% active material/g). Another product is a guinea pig liver transglutaminase, available from Sigma Chemical Company, having from 1.5-3 units/mg protein. As a guideline to formulation with this type of product, the weight percent concentrations of the Activa™ material range between about 0.5 to about 1.75% (about .004 to about .025% of transglutaminase) by weight of the total composition, with best results occurring between about .75 to about 1.25% (about .006 to about .017% transglutaminase), and optimum results occurring at about 1% (about .0086 to about .0135% of transglutaminase). It has been observed that higher amounts not only do not appear to enhance the effect, but may actually defeat the effect (see Example 3). Given these guidelines, it is well within the skill in the art to determine the optimum concentration of any given available transglutaminase product of different potency. It will also be understood that "retention-effective amount" shall refer to both an amount effective to retain curl in a keratinous material, as well as an amount effective to enhance existing curl or impart curl where none previously existed.

The transglutaminase may be formulated into any type of vehicle suitable for application to the hair or eyelashes, with the following guidelines. The optimum activity of transglutaminase is observed at a pH from about 5 to about 9, and a pH of about 6 to about 7 is particularly preferred. Heat and/or surfactants, particularly anionic surfactants, and particularly anionics in the presence of heat, may also affect the activity of the enzyme, so care should be taken in formulation to select surfactants that will not significantly alter the activity of the transglutaminase under the intended conditions of use. Given these guidelines, the transglutaminase can be readily formulated into a variety of product types, i.e., gels, creams, lotions, serums, emulsions, suspensions, or any type of topical delivery system that can be used for application to the hair or lashes. Methods and guidelines for formulation can be found, for example, in Harry's Cosmeticology, 8th edition, M. Reiger, Ed. 2000, the contents of which are incorporated herein by reference. For application to the hair, the product can be, for example, a styling product, a conditioner or a shampoo. For application to the lashes, the product can take the form of a lash treatment product, a lash primer, a lash topcoat, or a mascara, each of which may additionally contain pigment.

The compositions used in the method of the invention may also include other cosmetic adjuncts that are appropriate to the intended use of the composition. Such adjuncts are well known in the art, and examples of such can found in the International Cosmetic Ingredient Dictionary and Handbook, Ninth Edition, published by the Cosmetics, Toiletries and Fragrance Association; its contents are incorporated herein by reference. Examples of essentially inert materials that may be employed in the composition include, but are not limited to, emollients, such as various oils(silicone or hydrocarbon), fatty alcohols, esters, waxes and the like, film-forming agents such as cellulose, acrylic or acetate derivatives , thickeners and gellants, moisturizing agents, humectants, colorants, surfactants, particularly nonionic and cationic surfactants, and other cosmetically acceptable material. The compositions may also contain active materials appropriate to the use of the composition. Examples include skin (scalp) and hair conditioning agents, sunscreens, antiirritants, antiinflammatories, antimicrobials, hair growth enhancers, antioxidants, and the like.

The method of the present invention can be carried out in a number of ways. If applied as a hair conditioner, a styling product, or a shampoo, the product will be applied in the typical manner for application of such product. Although styling products, and some conditioners, may be left on the hair, typically conditioners and shampoos will be rinsed off the hair. However, because the transglutaminase is substantive to the hair, sufficient binding to the hair can occur in the normal application time, i.e., several minutes, for shampoos and conditioners. In addition, the reaction speed is enhanced, within limits, by exposure to higher temperatures (about 55°C being preferred), such as may be found with the use of a blow dryer (about 45°C) or shower water (about 60°C). Reaction speed can also be controlled by use of a formula having optimum pH, i.e., between about 6 and 7. When applied as a lash product, the product is obviously left on, and rinsing off is not an issue.

The benefit of the method can be appreciated in a variety of ways. The transglutaminase can be used in the treatment of curled hair to assist in curl retention in the presence of humidity and/or heat which would normally cause the curl to lengthen and droop. This method is applicable to hair which is curled naturally, chemically (i.e., permed) or mechanically (e.g.., by setting with rollers, curling iron, etc.). The product may be applied by the user as described above on an as-needed basis, e.g., during periods of high humidity, or it may be used on a daily basis to ensure curl retention, whatever the atmospheric conditions. The transglutaminase product can also be used to retain curl in eyelashes, which like hair can be susceptible to loss of curl under humid conditions; as with hair, this method can be used on eyelashes already having a natural curl, or on lashes that have been artificially curled, e.g., with an eyelash curler., by application to the lashes on an as-needed or as desired basis.

The transglutaminase acts on components that are normally present in all hair, regardless of its curl or lack thereof.

The invention is further illustrated by the following non-limiting examples.

### Example 1. The following formulas exemplify compositions useful in the method of the invention.

### A. "Tight" curl styling product

| Material | Weight % |
|---|---|
| Phase I | |
| <4a> | |
| Purified water | 80.90 |
| Glycerin | 2.00 |
| Panthenol | 0.10 |
| Hydrolyzed wheat protein/hydrolyzed wheat starch | 0.25 |

| Phase II | |
|---|---|
| Cetearyl alcohol/behentrimonium chloride | 4.00 |
| Cetyl alcohol | 2.50 |
| Phenyl trimethicone | 1.25 |
| Glycerin/water/sodium PCA/urea/trehalose/ Polyquaternium-51/sodium hyaluronate | 2.00 |
| | |

| Phase III | |
|---|---|
| Phenoxyethanol | 1.00 |
| | |

| Phase IV | |
|---|---|
| Transglutaminase/maltodextrin* | 1.00 |
| Purified water | 4.00 |
| Organomodified silicone polyether copolymer | 1.00 |

| | |
|---|---|
| *Activa™ TG-TI | |

### B. "Soft" curl styling product

| Material | Weight % |
|---|---|
| Phase I | |
| Purified water | 65.65 |
| Glycerin | 3.00 |
| Pantethine | 0.05 |
| PVP | 2.00 |
| | |

| Phase II | |
|---|---|
| Dimethicone | 1.50 |
| Cetearyl alcohol | 3.80 |
| Cetyl alochol | 3.00 |
| Petrolatum | 3.00 |
| Beeswax | 0.90 |
| Microcrystalline wax | 0.70 |
| Squalane | 1.00 |
| Isopropyl myristate | 2.00 |
| | |

| Phase III | |
|---|---|
| Polyquaternium chloride-7 | 2.50 |
| Cetrimonium chloride | 1.00 |
| Organomodified silicone polyether copolymer | 0.50 |
| | |

| Phase IV | |
|---|---|
| Panthenol | 0.50 |
| Panthenyl ethyl ether | 0.10 |
| Phytantriol | 0.10 |
| Glycerin/water/sodium PCA/urea/trehalose/ Polyquaternium-51/sodium hyaluronate | 3.00 |
| Phenyl trimethicone | 1.00 |
| Transglutaminase/maltodextrin* | 1.00 |
| Purified water | Q.S. |

| | |
|---|---|
| *Activa TG-TI | |

### C. Lash Curling Gel

| Material | Weight % |
|---|---|
| Gelcarin® GP 812 (Polysaccharide)* | 0.25 |
| Water | 15.00 |
| 5% Potassium chloride | .07 |
| | |
| Gelcarin® GP 379 (polysaccharide)* | .50 |
| Water | 18.00 |
| 10% Calcium Chloride | 0.20 |
| | |
| Bentone EW | 1.00 |
| Water | 25.00 |
| | |
| Transglutaminase | 0.01 |
| water | QS |

| | |
|---|---|
| *Carageenan(FMC Corporation) | |

### D. Curl Enhancing Conditioner

| Material | Weight % |
|---|---|
| Phase I | |
| Purified water | QS |

| Phase II | |
|---|---|
| Hydroxyethylcellulose | 0.25 |
| | |

| Phase III | |
|---|---|
| Caffeine | 0.05 |
| Panthenol | 0.10 |
| Aloe vera gel | 0.10 |
| | |

| Phase IV | |
|---|---|
| Cetyl alcohol | 6.00 |
| Cetearyl alcohol/behentrimonium chloride | 2.00 |
| Dimethicone | 2.00 |
| Linoleic acid | 0.10 |
| | |

| Phase V | |
|---|---|
| Stearalkonium chloride | 1.00 |
| | |

| Phase VI | |
|---|---|
| Cetyl octanoate | 0.05 |
| Caprylic/capric triglyceride | 0.75 |
| Potassium cholesterol sulfate | 0.10 |
| Ceramide | 0.10 |
| | |

| Phase VII | |
|---|---|
| Pantethine | 0.05 |
| Phytantriol | 0.10 |
| | |

| Phase VIII | |
|---|---|
| Hydrolyzed wheat protein/hydrolyzed wheat starch | 0.25 |
| Tocopherol acetate | 0.05 |
| Biosaccharide gum | 0.001 |
| Corn oil/Retinyl palmitate | 0.05 |
| Phospholipids | 0.05 |
| Panthenyl ethyl ether | 0.10 |
| Glycerin/water/sodium PCA/urea/trehalose/ Polyquaternium-52/sodium hyaluronate | 1.00 |

| Phase IX | |
|---|---|
| Transglutaminase/maltodextrin | 1.00 |
| Purified water | 4.00 |

### Example II

This example illustrates the use of transglutaminase in curl retention.

Compositions substantially identical with compositions A and B above are tested for their ability to cause curl retention in hair treated with them. The test was conducted as follows. Curly brown hair was purchased from De Meo Brothers. One gram of hair is the test sample size, and each sample tress is measured at its stretched-out length (length c). Each sample of hair is washed and dried. One gram of test product is applied to each, with the same products, minus the transglutaminase, being applied to other sample tresses as controls. The samples are dried (about 10-15 minutes) and placed in a humidity chamber at 90° relative humidity and 100°F. After 10 minutes, the samples are removed and measured again in its curled state (crest to crest), provided a base line of curl retention for all samples. The samples are returned to the chamber, and removed and measured again at 3 hours, and then again after 7 hours in the chamber. A curl factor is calculated, which is equal to the curled length divided by the length c. This figure represents the extent to which the curly hair resists lengthening in the presence of moisture, a lower number indicating a shorter curl length and thus better curl retention. The results showing the curl factor for each sample(average of 5 repetitions) are presented in Table 1.

**Table 1.**

| | **Time** | | |
|---|---|---|---|
| | 10 minutes | 3 hours | 7 hours |
| "Tight" (0% TG) | .857 | .9115 | .79 |
| "Tight" (1% TG) | .770 | .797 | .80 |
| "Soft" (0% TG) | .909 | .901 | .95 |
| "Soft: (1% TG) | .774 | .71 | .79 |

The results show that the samples treated with transglutaminase exhibit a better curl retention over a number of hours.

### Example 3: Dose response

Testing is done to determine the pattern of curl retention against concentration of transglutaminase. The testing is conducted using various concentration of the Activa™ product. The actual enzyme concentration in this product is discussed above, but the concentrations discussed here reflect the concentration of the commercial product containing transglutaminase and maltodextrin.

Hair samples as described in the previous example are exposed to concentrations of 0, 1%, 2%, 5% and 10% of the test product for a period of 30 minutes, and the curl factor determined. At 1%, there was no droop at all, and in fact a 16.6% increase in curl tightness. However at 2% there was a 25% droop, at 5%, a 33% droop, and at 10%, a 16% droop. The test is repeated with a 60 minute exposure. Again, at 1% curl is increased, by 25%, while droop is observed at 2%, 5% and 10% (16.6, 25 and 16.6% droop, respectively). This illustrates an unexpected result, that increased concentrations of transglutaminase not only do not result in curl retention, but actually result in curl droop.

## Claims

1. A method of retaining curl in a keratinous material which comprises applying to the keratinous material a composition containing a transglutaminase product in an amount ranging from 0.5% to 1.75% by weight, wherein said transglutaminase product is a combination of powdered microbial enzyme and maltodextrin containing 86-135 units of transglutaminase activity per gram.

2. The method of claim 1 in which the composition has a pH of 6 to 7.

3. The method of claim 1 in which the keratinous material is hair.

4. The method of claim 3 in which the application of the composition is followed by the application of heat.

5. The method of claim 1 in which the keratinous material is eyelashes.

6. The method of claim 1 in which the transglutaminase product is present in the composition in an amount of from 0.75% to 1.25% by weight.

7. A method of retaining curl in a keratinous material which comprises applying to the keratinous material a composition containing transglutaminase in an amount sufficient to provide the composition with 0.43 - 2.3625 units of TGase activity per gram of composition.

8. The method of claim 7 in which the amount of transglutaminase is sufficient to provide the composition with 0.645 - 1.6875 units of TGase activity per gram of composition.

## Patentansprüche

1. Verfahren das dazu dient, ein Keratinmaterial in gewellten Zustand zu halten, wobei eine Zusammensetzung, die ein Transglutaminaseprodukt in einer Menge im Bereich von 0,5 % bis 1,75 % nach Gewicht enthält, auf das Keratinmaterial angewendet wird, wobei es sich bei dem Transglutaminaseprodukt um eine Kombination aus pulverförmigem mikrobiellem Enzym und Maltodextrin handelt, welche pro Gramm eine Transglutaminase-Aktivität von 86 bis 135 Einheiten aufweist.

2. Verfahren nach Anspruch 1, wobei die Zusammensetzung einen pH-Wert von 6 bis 7 hat.

3. Verfahren nach Anspruch 1, wobei es sich bei dem Keratinmaterial um Haar handelt.

4. Verfahren nach Anspruch 3, wobei nach der Anwendung der Zusammensetzung eine Wärmeeinwirkung erfolgt.

5. Verfahren nach Anspruch 1, wobei es sich bei dem Keratinmaterial um Wimpern handelt.

6. Verfahren nach Anspruch 1, wobei das Transglutaminaseprodukt in der Zusammensetzung in einer Menge von 0,75 % bis 1,25 % nach Gewicht vorliegt.

7. Verfahren, das dazu dient, ein Keratinmaterial in gewelltem Zustand zu halten, wobei eine Zusammensetzung auf das Keratinmaterial aufgebracht wird, die Transglutaminase in einer Menge enthält, welche ausreicht, um der Zusammensetzung eine TGase-Aktivität von 0,43 bis 2,3625 Einheiten pro Gramm der Zusammensetzung zu verleihen.

8. Verfahren nach Anspruch 7, wobei die Menge an Transglutaminase ausreicht, um der Zusammensetzung eine TGase-Aktivität von 0,645 bis 1,6875 Einheiten pro Gramm der Zusammensetzung zu verleihen.

## Revendications

1. Procédé destiné à maintenir des boucles dans une matière kératinique qui comprend l'application à la matière kératinique d'une composition contenant un produit de transglutaminase dans une quantité allant de 0,5% à 1,75% en poids, dans lequel ledit produit de transglutaminase est une combinaison d'enzyme microbienne en poudre et de la maltodextrine contenant 86-135 unités d'activité de transglutaminase par gramme.

2. Procédé selon la revendication 1, dans lequel la composition a un pH de 6 à 7.

3. Procédé selon la revendication 1, dans lequel la matière kératinique est les cheveux.

4. Procédé selon la revendication 3, dans lequel l'application de la composition est suivie par l'application de la chaleur.

5. Procédé selon la revendication 1, dans lequel la matière kératinique est les cils.

6. Procédé selon la revendication 1, dans lequel le produit de transglutaminase est présent dans la composition en une quantité de 0,75% à 1,25% en poids.

7. Procédé destiné à maintenir des boucles dans une matière kératinique qui comprend l'application à la matière kératinique d'une composition contenant un produit de transglutaminase dans une quantité suffisante pour conférer à la composition 0,43 - 2,3625 unités d'activité TGase par gramme de composition.

8. Procédé selon la revendication 7, dans lequel la quantité de transglutaminase est suffisante pour conférer à la composition entre 0.645 et 1,6875 unités d'activité TGase par gramme de composition.
